# EUROPEAN PATENT APPLICATION

(11) **EP 2 813 213 A1**
(43) Date of publication of application: **17.12.2014**
(21) Application number: 13171697.9
(22) Date of filing: 12.06.2013
(51) Int. Cl.: A61K 9/00, A61K 47/34, A61K 9/51

(54) **Injectable hydrogel composition, method for the preparation and uses thereof**

(71) Applicant: Nederlandse Organisatie voor Toegepast -Natuurwetenschappelijk Onderzoek TNO, 2628 VK Delft (NL)
(72) Inventor: Papen-Botterhuis, Nicole Ellen, 2628 VK Delft (NL); van Ee, Renz, 2628 VK Delft (NL); Craenmehr, Eric Gerardus Maria, 2628 VK Delft (NL); Timmer, Klaas, 2628 VK Delft (NL)
(74) Representative: Jansen, Cornelis Marinus

(57) **Abstract**

The present invention provides an injectable hydrogel composition comprising an aqueous dispersion of inorganic nanoparticles linked to a block copolymer by means of a functional group that is present in the copolymer, wherein said block copolymer has a lower critical solution temperature (LCST) in an aqueous medium, wherein the block copolymer comprises a thermoresponsive polymer block and an osmotically active polymer block and wherein the number average molecular weight of the block copolymer is at least 2 kDa. The hydrogel composition according to the invention has a low viscosity below the LCST and forms a gel by heating to a temperature above the LCST of the block copolymer.

The described hydrogels can be used in the sustained release of medicaments, tissue engineering, release agent of osmotic polymers and other medical and non-medical applications.

## Description

The invention relates to hydrogel compositions useful, for example, in biomedical applications. Particularly, the invention relates to an injectable hydrogel composition, a method for the preparation thereof, a method for the preparation of a gel therefrom, a gel obtained therefrom and the uses thereof.

Hydrogels are three-dimensional hydrophilic networks that can absorb and retain a considerable amount of water. Hydrogels have been widely used in the medicine such as in drug delivery, tissue engineering and as a release agent for osmotic polymers. Injectable hydrogels are of particular interest because drugs, proteins, and cells can be easily incorporated into polymer solutions prior to administration. For the insertion of the gels into the body, minimal invasive surgery is required since the gels are administered by simple injection. After insertion, under the influence of physiological conditions, the hydrogels rapidly convert into a gel.

For use in drug delivery and tissue engineering, hydrogels should be low-viscosity solutions prior to injection, and should rapidly gel in the human body. And hydrogels are of particular interest when they have a water content similar to that of tissues.

Many existing materials for injectable hydrogels cannot provide for an osmotic pressure and thus shrink in an osmotically active environment.

Hydrogels that are osmotically active are polymeric networks that can absorb and retain a considerable amount of water while maintaining shape. They exist usually in a solid form of a prosthesis or in a gelled form, which forms are both not suitable to be injected. An example of such a hydrogel is described in US-A-2012/0143338, wherein a polyvinyl alcohol copolymer is used as a solid body to replace or supplement an intervertebral disc.

Injectable osmotic active hydrogels are known, for example, from E. Rederstorff et al., Acta Biomaterialia 7(2011) 2119-2130 and from F. Horkay et al., Polymer 51 (2010) 4424-4430. However, the described hydrogels are gelled by a chemical cross-linking reaction involving the condensation of functional groups. This has certain disadvantages when used in a body such as necessity of performing a chemical reaction in the body and a limited time for the reaction. Performing a chemical reaction in the body can lead to by-reactions or side reactions with substances occurring in the body and/or with drugs, cells, proteins that are also present in the gel, which is an important disadvantage.

It is therefore desired to provide a hydrogel composition which has a low viscosity (injectable) before use and which gels into an osmotically active gel induced by temperature change, e.g. when it is injected into a body, wherein gelling is induced by temperature only and with no or hardly any chemical reactions.

In order to address one or more of the foregoing desires, the present invention provides, in one aspect, an injectable hydrogel composition comprising an aqueous dispersion of inorganic nanoparticles linked to a block copolymer by means of a functional group that is present in the copolymer, wherein said block copolymer has a lower critical solution temperature (LCST) in an aqueous medium, wherein the block copolymer comprises a thermoresponsive polymer block and an osmotically active polymer block and wherein the number average molecular weight of the block copolymer is at least 2000 Dalton (2 kDa).

In another aspect, the invention provides a method for the preparation of the hydrogel composition according to the invention, comprising combining the inorganic nanoparticles with the block copolymer in an aqueous medium at a temperature lower than the LCST of the copolymer.

In a further aspect, the invention provides a method for the preparation of an osmotically active gel, comprising heating the hydrogel composition according to the invention to a temperature higher than the LCST of the copolymer.

In yet a further aspect, the invention provides a gel obtained by the heating of the hydrogel composition according to the invention to a temperature above the LCST of the block copolymer.

Finally, the invention provides the use of the hydrogel composition or the gel according to the invention, in the sustained release of medicaments, tissue engineering and as a release agent of osmotic polymers. Other uses can be in the provision of injectable absorbent materials such as super absorbers for water.

The present invention is based on the judicial insight that coupling of a copolymer having thermoresponsive polymer blocks and osmotically active polymer blocks with inorganic particles can provide a hydrogel with the desired properties of injectability at lower temperatures and forming of an osmotically active gel at higher temperatures. "Osmotically active" gel means it attracts water and swells in demi water. In other words, an osmotically active gel is in osmotic equilibrium with the osmotically active environment (has the same osmotic pressure).

The material according to the invention is an injectable hydrogel composition. Hydrogel composition means a composition that is able to form a gel in an aqueous medium (hydrogel) under certain conditions, or better under the conditions required for the intended use. "Injectable" means here a free-flowing, or an easy flowing liquid. Particularly, an injectable liquid has a sufficiently low viscosity to be introduced via a syringe, such as a syringe of 26 gauge. For intradiscal surgery typical a syringe of 29 gauge is used. The composition according to the invention has preferably a sufficiently low viscosity to be introduced via a syringe of 32 gauge.

The hydrogel composition comprises an aqueous dispersion of inorganic nanoparticles modified with a block copolymer. "Nanoparticles" generally mean particles having at least one particle size dimension smaller than 100 nm. "Aqueous" means that the liquid present in the dispersion contains water. This however does not exclude that other solvents may be present. The hydrogel composition can also comprise other solvents, preferably miscible with water. Suitable solvents are selected from the group consisting of ethylene glycol, methanol, ethanol, propanol, isopropanol, butanol, tert-butanol, formic acid, acetic acid, dimethylformamide, dimethylsulfoxide, acetone, tetrahydrofuran and dioxane. The hydrogel can also comprise salts and other additives. Examples of salts are NaCl, phosphate salts (such as PBS), CaCl₂, sodium dodecyl sulphate, and so on.

The block copolymer used in the present invention possesses a lower critical solution temperature (LCST) in an aqueous medium. As will be understood by the skilled person, at a temperature below the LCST the polymer displays hydrophilic properties as a result of which the aqueous medium with the polymer will be a transparent liquid. At a temperature similar to or above the LCST the polymer will display hydrophobic properties as a result of which the aqueous medium will be an opaque liquid (that is, hazy to white) due to the (partial) precipitation of the LCST polymer.

In the present invention, the inorganic nanoparticles are modified by the block copolymer. Such a combination of two materials provides for the gelation at the temperatures above the LCST. Due to the binding of the polymer to the inorganic particles it is believed that the gelling, without wishing to be bound by any theory, is caused by forming a physical entangled network above LCST.

The hydrogel composition according to the present invention exhibits thermo-induced gelation. This means in the present invention that the liquid hydrogel composition becomes a gel when the temperature of the composition is increased above the LCST. In some embodiments, it can be desired that the thermo-induced gelation is reversible. "Reversible" means that the composition turns into a liquid again when the temperature of the composition is decreased below the LCST. The reversibility can, for example, be tuned by the use of functional groups in the polymer chains.

The LCST of the copolymer can vary from -40 to 300°C, preferably, in the range from -20 to 100°C. If the hydrogel of the invention is intended to be used in a human or animal body, the copolymer preferably has the LCST in aqueous solutions in the range 20-45°C. That means that before the injection, the hydrogel is liquid and injectable and after the injection, the hydrogel gels.

Particularly, the complex shear modulus G* of the hydrogel is preferably in the range 1-100 Pa below the LCST, measured using an Anton Paar MCR301 Rheometer at a strain of 0.5% and a frequency of 1Hz. The complex shear modulus G* measured above the LCST and further the same conditions, is preferably at least 100, and more preferably in the range 1000-50,000 Pa. The change in complex shear modulus is preferably at least 1 decade (factor of 10 difference), more preferably 2 or 3 decades (factor of 100 or 1000 difference, respectively).

In case the hydrogel of the invention is intended for use in a human or animal body, the hydrogel and all its components should be biocompatible. In some cases, it may be desired that the hydrogel is biodegradable.

The hydrogel according to the invention comprises inorganic nanoparticles modified by the block copolymer. The presence of inorganic particles is necessary for the gelation properties of the resulting hydrogel; without the nanoparticles, no gelation of the copolymer is observed but only precipitation from the aqueous medium. The inorganic nanoparticles can also be suitably used as a delivery vehicle for the active ingredients that can be loaded in the hydrogel.

The particles used in accordance with the present invention are derived from an inorganic material. Suitably, the inorganic material can be selected from the group consisting of minerals, silicates, metal oxides, natural and synthetic clays, and layered double hydroxides.

In some embodiments, the inorganic material may comprise the natural or synthetic clays smectite, laponite, bentonite, hectorite, saponite, vermiculite, chlorite, sepiolite, palygorsite or magadiite. More preferably, the inorganic material comprises laponite, bentonite, sepiolite or hectorite.

Preferably, the nanoparticles comprise layered double hydroxides (LDHs). The advantage of using LDHs is that these are particularly suitable to increase cellular uptake (transfection) and especially as a gene delivery vehicle. Furthermore, the LDH's are biodegradable causing controlled release of added agents.

LDHs are a group of anion-exchanging materials containing mixed metal hydroxides similar to brucite, Mg(OH)₂. In a brucite layer, each Mg²⁺ ion is octahedrally surrounded by six OH- ions and the different octahedra share edges to form an infinite 2D layer. In hydrotalcite [Mg₆Al₂(OH)₁₆][CO₃ ·4H₂O], the replacement of Mg²⁺ by Al³⁺ gives the brucite-like layer positive charges that are balanced by carbonate anions located in the inter-layer region. The positively charge brucite-like layers ([Mg₆Al₂(OH)₁₆]²⁺), interlayer anions as well as water molecules ([CO₃ ·4H₂O]²⁻) are held together via electrostatic interactions and hydrogen bonds to form a 3D structure.

In general, LDHs can be chemically expressed by a formula like [Q²⁺ₗ₋ₓR³⁺ₓ(OH)₂]^{x+}[(Z^{m-})_{x/m} ·nH₂O]^{x-}, where Q²⁺ can be most divalent cations such as Mg, Zn, Ni, Co, and Fe, R³⁺ most trivalent cations such as Al, Fe, and Cr, and Z^{m-} represents exchangeable anions, such as CO₃²⁻, Cl⁻, SO₄²⁻, various organic anions and complex anions. In some application, it is desired that Q is Mg and R is Fe.

LDHs can be readily synthesized in the laboratory by methods conventionally known in the art. The commonly used method is coprecipitation at varied or constant pH, followed by aging at a certain temperature.

The particles of the inorganic material to be used in accordance with the present invention suitably have an average largest dimension in the range 10 nm - 5 µm, preferably in the range 20 nm - 2 µm. The particle size is measured by transmission electron microscopy (TEM).

The inorganic nanoparticles used in the present invention for loading of active ingredients, are modified with the block copolymer. In particular, the modification results in that the inorganic particles become linked to the polymer chains of the block copolymer by means of a functional group that is present in the polymer chains. Such a functional group can, for example, be present in the thermoresponsive block and/or in the osmotically active block. The linkage between the copolymer and the inorganic particles can be physical or chemical in nature, e.g. an ionic, covalent or coordinative linkage or hydrogen bonding.

The functional group(s) present in the polymer chains can suitably be chosen from the group consisting of hydroxyl, amine, epoxide, acid, anhydride, ammonium, carboxylate, sulphate, sulphonate, sulphinate, silane, phosphate, phosphonate, phosphinate, phosphite, phosphinite, sulphide, disulphide, maleic acid, phosphine, phosphine oxide, phosphonium, sulphonium, oxonium, ether, aldehyde and combinations of these.

In some embodiments, it is preferred that the linkage is ionic. This means that the nanoparticles and the functional group in the polymer chains are linked by the interaction between opposite charges. For example, when inorganic nanoparticles with a negatively charge (e.g. clays) are used, the copolymer should contain a positively charged functional group (e.g. amine group). In case positively charged nanoparticles are used (e.g. LDHs), the copolymer should contain a negatively charged functional group (e.g. carboxylic acid group). Ionic linkage is preferred because the reaction can be carried out very easily by ionic exchange and does not require additional reagents for the reaction to take place.

Any suitable method known in the art can be used to perform the linking of the particles to the copolymer. For example, in case of ionic linkage, the charged inorganic nanoparticles can be combined with a copolymer having a functional group with the opposite charge, in an aqueous medium at a temperature lower than the LCST of the copolymer, and are allowed to react during a certain time period, for example, 8 hours or overnight. Suitable reaction conditions are known to the skilled person or can be obtained by routine experimentation.

The block copolymer used in the present invention comprises a thermoresponsive polymer block and an osmotically active polymer block. Under "thermoresponsive polymer" in the present case is understood a polymer having a LCST. In combination with the nanoparticles, the resulting composition has the advantageous properties of low viscosity at the temperatures lower than the LCST, gelling at higher temperatures and osmotic activity of the gel.

The thermoresponsive polymer block contributes to the thermoresponsive properties of the copolymer, that is, that the resulting copolymer possesses a LCST. The precise value of LCST depends on several factors, including the weight ratio of the two blocks in the copolymer.

The use of a block copolymer is advantageous because as found by the inventors, it appears to possess the properties of the both blocks without disturbing the interaction of the polymer with the inorganic particles. The two individual polymers were found to not to mix at higher temperatures, with two individual polymers an inhomogeneous mixture obtained which sags out. Since the resulting gelling properties depend on the complex interaction of the nanoparticles with the polymer chains, it could not been foreseen that the modification of the polymer chains, in this case by adding an osmotically active block, would preserve the gelling properties. It is the insight totally attributed to the inventors that such combination of the properties is realized in the hydrogel composition according to the invention.

The thermoresponsive polymer block can suitably be selected from the group consisting of thermoresponsive homo- and co-polymers of acrylamides, methacrylamides, methacrylic acid, hydroxyalkylacrylates, hydroxyalkylmethacrylates, allylamine, oligo(ethylene glycol)methacrylates and N-vinyl-N,N-disubstituted amides (such as N-vinylcaprolactam or vinyl ethylimidazole), polymers and copolymers of ethylene glycol and propylene glycol, copolymers of poly ethylene glycol and polyesters, surfactants containing oligo(ethylene glycol), polymers and copolymers of vinyl acetate and vinyl alcohol, poly amino acids, chitosan derivatives and cellulose derivatives.

In biomedical applications, the thermoresponsive polymer block preferably has the lower critical solution temperature in aqueous solutions under 45°C and preferably above 20°C, yet more preferably above 25°C. In a most preferred embodiment, the thermoresponsive block is selected from the group consisting of poly(N-isopropylacrylamide) (the LCST about 32°C), poly(vinylcaprolactame) (the LCST about 32°C), poly(3-ethyl-N-vinyl-2-pyrrolidone) (the LCST about 26°C), polyvinyl methyl ether (the LCST about 35°C) and mixtures thereof.

The osmotically active polymer used to provide the osmotic activity to the gel, can be an anionic, a non-ionic or a cationic polymer.

In some embodiments, the osmotically active polymer block comprises an anionic polymer, that is, a negatively charged polymer. In other embodiments, the osmotically active polymer block is not charged (non-ionic polymer). The osmotically active polymer block can also be a cationic polymer such as polyethyleneimine, positively charged polyamino acids (like polylysine), positively charged polydextrine.

Preferably, the osmotically active polymer is selected from the group consisting of a cellulose derivative, a polysaccharide, polyethylene glycol, a derivative of an sulfonic acid polymer, or combinations thereof. More preferably, the osmotically active polymer is selected from the group consisting of cellulose acetates, ethyl cellulose, hydroxypropylmethylcellulose, hyaluronic acid polymer, 2-acrylamido-2-methylpropanesulfonic acid polymer (pAMPS; generally synthesized using AMPS salts such as the sodium sulfonates), polyethyleneimine, positively charged polyamino acids, positively charged polydextrine and combinations thereof.

It is preferred for the gelling properties that the block copolymer used in the present invention has a number average molecular weight of at least 2 kDa, preferably at least 5 kDa, more preferably at least 10 kDa. In case pAMPS and pNIPAAM are used as the blocks in the block copolymer, the number average molecular weight should be at least 20 kDa, preferably at least 30 kDa, more preferably at least 45 kDa. The maximum weight is not limited since the gelling always takes place at higher lengths of the polymer chains.

For example, when pAMPS and pNIPAAM are used as the blocks in the block copolymer, and especially in a molar ratio of 20 to 80, it was found by the inventors that in order to have the same osmotic pressure as in the natural nucleus pulposus material, the best suited concentrations is 16-22 wt.% of the total solid weight of the hydrogel formulation. The mentioned values include both the content of the polymers present in the co-polymer and the content of individual polymers (either pAMPS or pNIPAAM) that may be present as a homopolymer in the dispersion. Addition of individual polymers may be desired to achieve the optimal concentration (for the desired osmotic pressure) of the osmotically active polymer in the dispersion. In this way, the osmotic activity can be tuned *in situ* without the need to synthesize a co-polymer with a different weight ratio of the polymer blocks. Therefore, in some embodiments, the composition according to the invention further comprises the thermosensitive polymer and/or the osmotically active polymer. These polymers are then present in the mixture individually and not in the form of a copolymer. Individual polymers can be added to the composition before or after the reaction between the nanoparticles and the polymer chains. A skilled person is able to determine necessary concentrations and conditions in order to arrive to a desired osmotic pressure.

The hydrogel of the invention preferably comprises water in an amount of at least 50 wt.%, more preferably 60-99 wt.%, based on total composition.

The hydrogel according to the invention further preferably comprises a biologically active ingredient, such as drugs, proteins, cells, nucleotides, oligonucleotides, genes, etc.

The hydrogel composition according to the invention can be obtained by a method comprising combining the inorganic nanoparticles with the block copolymer in an aqueous medium at a temperature lower than the LCST of the copolymer. After combining, the polymers and particles are allowed to react in order to bind to each other. Typically, a suitable amount of time is several hours, for example for 8 hours or overnight. If desired, the nanoparticles and/or the copolymer can be provided in the form of an aqueous dispersion or solution.

It is particularly preferred to combine the already prepared (or otherwise provided) block copolymer and the nanoparticles. *In-situ* polymerization of the copolymer should be avoided. *In-situ* polymerization of the block copolymer in the presence of the nanoparticles can lead to the crosslinking between the nanoparticles and the polymer chains, which in turn results in that the hydrogel composition is not injectable or lacks thermo-induced gelation properties.

The invention further provides a gel (hydrogel) obtained by heating the hydrogel composition according to the invention to a temperature above the LCST of the block copolymer.

The gel according to the invention is osmotically active. The osmotic activity of the gel can be tuned by the ratio of the LCST polymer block to the osmotically active polymer block. This can be desired in biomedical applications because different parts of the body have different osmotic pressures like subcutaneous, intravenous, intramuscular or intradiscal. The weight ratio to provide the desired the osmotic activity is dependent on the particular polymers used as the LCST block and the osmotically active polymer block. To determine the suitable level of osmotic activity a skilled person can determine suitable weight ratios by routine experimentation.

The hydrogels according to the present invention, can widely be used, for example, in the sustained release of medicaments (drug delivery), tissue engineering and as a release agent for osmotic polymers. When used for drug delivery, the present hydrogels have an advantages that, because the shape and the physical properties of the gel remain maintained in the osmotically active environment, no burst release takes place, which usually happens with non-osmotically active gels that shrink after injection.

The hydrogels can also be used in other applications such as wound care, contact lenses, enhanced oil recovery, culture medium for cell culturing, fire prevention, etc.

The following non-limiting examples illustrate the invention and do not limit its scope in any way. In the examples and throughout this specification, all percentages, parts and ratios are by weight unless indicated otherwise.

### Example 1

### Synthesis of NaAMPS

As RAFT agents are susceptible to hydrolysis under strong acidic or basic conditions, the AMPS monomer [2-acrylamido-2-methyl-1-propanesulfonic acid] was used for polymerisation in the form of a sodium salt NaAMPS. The salt NaAMPS was prepared in DMF in quantitate yield by reaction of commercial available NaOMe in MeOH in the presence of benzoquinone. Benzoquinone acts as inhibitor as well as indicator when the NaOMe in MeOH is added to the acidic monomer. The product is purified by precipitation in diethyl ether and characterized by ¹H NMR.

### Example 2

### Synthesis of pNaAMPS

RAFT synthesis (Reversible Addition-Fragmentation Chain Transfer) was used to prepare the blocks for the block copolymer poly(NIPAAM-block-AMPS). NIPAAM stands for N-isopropylacrylamide, AMPS stands for 2-acrylamido-2-methylpropanesulfonic acid.

The synthesis of pNaAMPS block by means of the RAFT process is performed with C₆H₅-C(S)-S-C(CH₃)(CN)CH₂CH₂COOH used as the RAFT agent in the presence of AIBN as initiator. Two attempts were made to prepare pNaAMPS.

The first attempt to prepare pNaAMPS was made using the acidic monomer after *in situ* neutralization with NaOH. The preparation of pNaAMPS was done in water and some ethanol had to be added in order to dissolve the RAFT agent. After stirring for 32 hours at 70 °C a pink product could be isolated after purification (precipitation in ethanol) in 65% yield, while the ¹H NMR spectrum clearly shows the presence of the aromatic protons of the RAFT agent. GPC analysis gives a Mw of 13600 g/mol and a PD (polydispersity) of 1.14 [GPC method: 0.1 m NaNO₃ in H₂O/CH₃CN (80:20 v/v)] at an aimed Mw of 14000 g/mol.

Secondly pNaAMPS was prepared using the NaAMPS described in example 1. The preparation of pNaAMPS was done in MeOH and a trace of water. After stirring for 32 hours at 70 °C a pink product is isolated after purification (precipitation in ethanol) in 67% yield. The ¹H NMR spectrum of this product clearly shows the presence of the aromatic protons of the RAFT agent. GPC analysis gives a Mw of 7200 g/mol and a PD of 1.14 [GPC method: 0.1 m NaNO₃ in H₂O/CH₃CN (80:20 v/v)] at an aimed Mw of 7150 g/mol.

### Example 3

### Synthesis of pNIPAAM-block-pNaAMPS

When preparing pNIPAAM-block-pNaAMPS copolymers by the RAFT process, it was started with a pNaAMPS-block and then the polymerization proceeded with the monomer NIPAAM. This approach (instead of starting with a block of NIPAAM) appeared to have an advantage of easier purification, that is the easier removal of remaining NIPAAM as compared with the removal of remaining NaAMPS. This is a consequence of the quite large difference in (non)solubility in organic solvents of (p)NIPAAM and (p)NaAMPS.

Two products pNIPAAM-block-pNaAMPS copolymers were synthesized, starting with a pNaAMPS-block. The first started with a pNaAMPS-block of Mw 13600, with NIPAAM as the second monomer and AIBN as initiator. The polymerization is performed in a mixture of MeOH and water (25:1). After 12 hours stirring at 70°C the reaction mixture is cooled to room temperature and the product is precipitated in diethyl ether.

The other product is synthesized from a pNaAMPS-block of Mw 7200, in a mixture of DMF and H₂O (20:3) as the solvent and AIBN as initiator. After 24 hours stirring at 70°C the reaction mixture is cooled to room temperature and the product is precipitated in aceton. Both products have a yield of 75% conversion and the resulting molar ratios NIPAAM/NaAMPS are 80/20 at an aimed ratio of 85/15, as determined by ¹H NMR and summarized in the Table 1. The Mw and PD of the products are determined by GPC (method MeOH-0.2m LiClO₄) and summarized in the Table 1 as well.

**Table 1 Characteristics of the synthesized copolymers**

| Mw NaAMPS starting block | Molar ratio NIPAAM/NaAMPS | Mw block copolymer | PD | yield % |
|---|---|---|---|---|
| 13600 | 80 : 20 | 46420 | 1.60 | 73 |
| 7200 | 79 : 21 | 18940 | 1.60 | 75 |

### Example 4

### Preparation of LDH hydrogel with pNIPAAM-block-pNaAMPS co-polymers

The two purified pNIPAAM-block-pNaAMPS copolymers prepared in Example 3 are dissolved in demi water, to which a concentrated (7.25 wt.%) dispersion of MgFe LDH particles is added.

Table 2 shows the details of the preparation of LDH gel formulation containing block copolymers of pAMPS-pNIPAAM.

**Table 2 Details of the preparation of LDH gel formulation**

| Mw NaAMPS starting block | pNIPAAM-block-pNaAMPS copolymer (g) | LDH (7.25 wt%) suspension (g) | Water (g) | Calculated solid weight content (wt.%) |
|---|---|---|---|---|
| 13600 | 3.16 | 5.80 | 4.79 | 23.7 |
| 7200 | 1.58 | 2.92 | 2.97 | 21.6 |

The dispersions are homogenized with the aid of a vortex. The samples are placed on a roller apparatus overnight to allow the LDH particles bind to the polymers.

### Example 5

### Osmotic properties of LDH hydrogels with pNIPAAM-pAMPS block copolymers

The osmotic tests are done using a PBS solution with 14wt.% PEG dissolved (PBS/PEG14wt.% solution) serving as a reference solution having the same osmotic pressure as the natural nucleus pulposus material.

The osmotic activity is measured using determination of solids. For that, the hydrogel solution was injected in a Slide-a-lyzer dialysis cassettes (Thermo Scientific), 0.5-3ml, 3.5KD MWCO (prewetted in PBS solution for minimal 5 minutes) following the standard protocol supplied with the Slide-a-lyzer. The dialysis cassettes were placed in PBS/PEG14 wt.% solution under stirring for 3 nights. After 3 nights the cassettes were removed from the solution and the sample removed from the cassettes following the Thermo scientific protocol. The sample was dried in an oven at 80°C to obtain its solid weight content.

When only pNIPAAM and no pNIPAAM-block-pNaAMPS copolymer was added to the LDH gel, shrinkage of the gel was seen within minutes in PBS/PEG14wt.% solution which increased in time until almost no water remained in the dialysis cassette.

For both LDH gel formulations with the pNIPAAM-block-pNaAMPS copolymer from example 3 swelling occurs and the solid content weight is decreased after the osmotic test in the dialysis cassette, as summarized in the Table 3.

**Table 3 Total solid weight content of the hydrogel formulations**

| Mw NaAMPS | Measured solid weight | Measured solid weight |
|---|---|---|
| 13600 | 24.4 (+/- 0.2) | 19.5 (+/- 1.0) |

| starting block | content (wt.%) before osmotic test | content (wt.%) after osmotic test |
|---|---|---|
| 7200 | 22.7 (+/- 0.2) | 18.3 (+/- 0.4) |

This means the uptake of water from the PBS/PEG14 wt.%, and thus the LDH gel formulations have a higher osmotic pressure than the PBS/PEG14 wt.% solution.

### Example 6

### Rheological properties of LDH hydrogels with pNIPAAM-pAMPS block copolymers

Finally, the rheological properties of the hydrogels with the pNIPAAM-pAMPS block copolymers of example 3 were tested by measuring the complex shear modulus G* using plate-to-plate setup at a strain of 0.5% and a frequency of 1 Hz using Anton Paar MCR301 Rheometer.

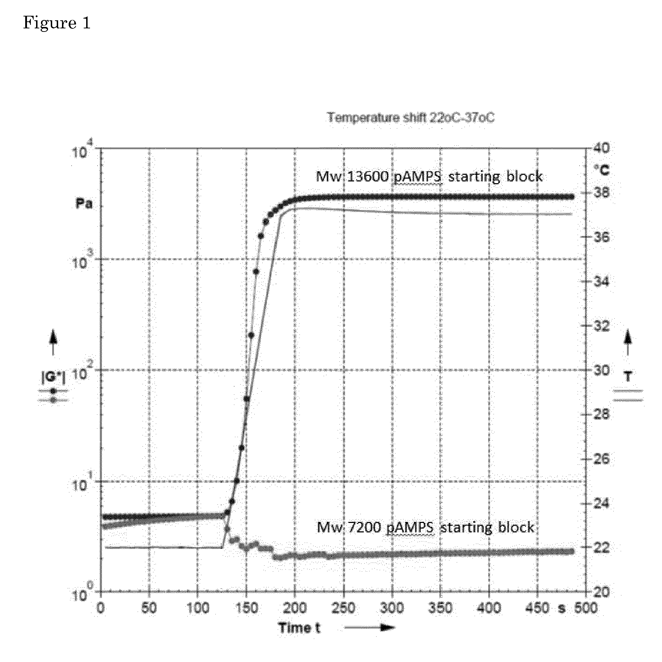

Figure 1 shows the change of the complex modulus of the gel in time while increasing the temperature from 22 to 37°C. From Figure 1 it can be seen that length differences of the polymer chains have a considerable impact on the viscosity switch in the hydrogel. For the shorter polymer (with Mw 7200 pAMPS starting block), no switch is observed at all, while for the longer polymer (with Mw 13600 pAMPS starting block), the switch is about 1000 fold and the final complex modulus reaches a value of 3 kPa.

It is also observed in Figure 1 that the LCST of the polymer has dropped to 23°C, leading to an immediate increase in complex modulus once the temperature starts rising above 22°C. For the application of the gel, this does not seem problematic, since the gels can be stored at lower temperature prior to injection, and usually ORs are air conditioned between 20 and 22°C.

### Example 7

### Swelling pressure of LDH hydrogels with pNIPAAM-pAMPS block copolymers

The swelling pressure is measured on a Zwick tensile testing machine with a confined compression setup in a PBS solution at 37 °C. The swelling pressure is the pressure that results from the osmotic activity in the gel when the gel is in a physiological solution. If the volume of the gel is kept constant whilst the gel is introduced to a PBS solution, the swelling pressure, Psw, can be measured. When the preload of 0.01N at 0.5 mm/s is reached, a 1% strain load was used to ensure the sample is loaded. The sample is allowed to swell for about two hours in which the load is recorded. The swelling pressure can be found as the maximum pressure after two hours.

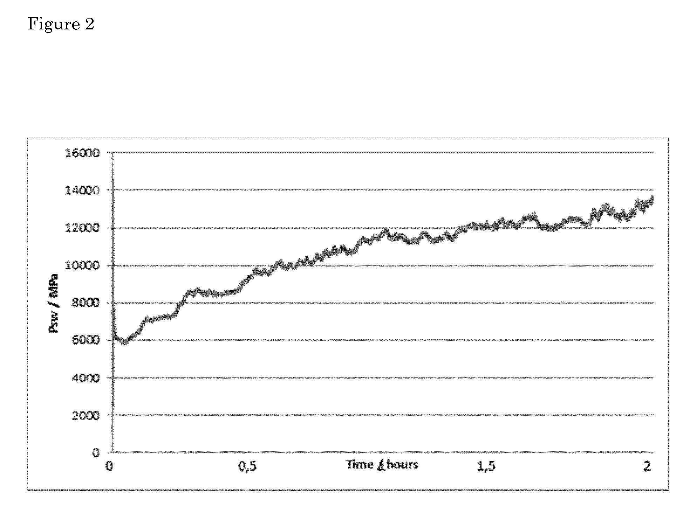

A swelling pressure Psw of 13.41 MPa is obtained for the hydrogel with the pNIPAAM-pAMPS block copolymer (with Mw 13600 pAMPS starting block) of example 3. Figure 2 shows the time-stress curve of the measurement.

## Claims

1. An injectable hydrogel composition comprising an aqueous dispersion of inorganic nanoparticles linked to a block copolymer by means of a functional group that is present in the copolymer, wherein said block copolymer has a lower critical solution temperature (LCST) in an aqueous medium, wherein the block copolymer comprises a thermoresponsive polymer block and an osmotically active polymer block and wherein the number average molecular weight of the block copolymer is at least 2 kDa.

2. The composition according to any one of the preceding claims, wherein the number average molecular weight of the block copolymer is at least 20 kDa, preferably at least 30 kDa, more preferably at least 45 kDa.

3. The composition according to claim 1 or 2, wherein the thermoresponsive polymer block is selected from the group consisting of polymers and copolymers of acrylamides, methacrylamides, methacrylic acid, hydroxyalkylacrylates, hydroxyalkylmethacrylates, allylamine, oligo(ethylene glycol)methacrylates and N-vinyl-N,N-disubstituted amides, polymers and copolymers of ethylene glycol and propylene glycol, copolymers of poly ethylene glycol and polyesters, oligo(ethylene glycol), polymers and copolymers of vinyl acetate and vinyl alcohol, poly amino acids, chitosan derivatives and cellulose derivatives.

4. The composition according to claim 3, wherein the thermoresponsive polymer is selected from the group consisting of poly(N-isopropylacrylamide) (pNIPAAM), poly(vinylcaprolactame), poly(3-ethyl-N-vinyl-2-pyrrolidone), polyvinyl methyl ether and mixtures thereof.

5. The composition according to any one of the preceding claims, wherein the osmotically active polymer is selected from the group consisting of a cellulose derivative, a polysaccharide, polyethylene glycol, a derivative of an sulfonic acid polymer, or combinations thereof.

6. The composition according to claim 5, wherein the osmotically active polymer is selected from the group consisting of cellulose acetates, ethyl cellulose, hydroxypropylmethylcellulose, hyaluronic acid polymer, 2-acrylamido-2-methylpropanesulfonic acid polymer (pAMPS and the sulfonate salts thereof), polyethyleneimine, positive polyamino acids, positive polydextrine and combinations thereof.

7. The composition according to any one of the preceding claims, wherein the inorganic material of the nanoparticles is selected from the group consisting of minerals, silicates, metal oxides, natural and synthetic clays, and layered double hydroxides.

8. The composition according to claim 7, wherein the clay is selected from the group consisting of smectite, laponite, bentonite, hectorite, saponite, vermiculite, chlorite, sepiolite, palygorsite or magadiite.

9. The composition according to claim 7, wherein the layered double hydroxides are can be chemically expressed by a formula [Q²⁺ₗ₋ₓR³⁺ₓ(OH)₂]^{x+}[(Z^{m-})_{x/m} ·nH₂O]^{x-}, where Q²⁺ is a divalent cation selected from the group consisting of Mg, Zn, Ni, Co and Fe, R³⁺ is a trivalent cation selected from the group consisting of Al, Fe, and Cr, and Z^{m-} represents exchangeable anions selected from the group consisting of CO₃²⁻, Cl-, SO₄²⁻, various organic anions and complex anions.

10. The compositions according to any one of the preceding claims, wherein the difference between the complex shear modulus of the copolymer below and above the LCST is at least 1 decade, and preferably 2 or 3 decades.

11. The composition according to any one of the preceding claims, having a complex shear modulus G* in the range 1-100 Pa, measured below the LCST, at a strain of 0.5% and a frequency of 1 Hz.

12. The composition according to any one of the preceding claims, having a complex shear modulus G* of at least 100 Pa, and preferably in the range 1000-50,000 Pa, measured above the LCST at a strain of 0.5% and a frequency of 1 Hz.

13. The composition according to any one of the preceding claims, wherein the LCST of the block copolymer is in the range 20-45°C.

14. The composition according to any one of the preceding claims, wherein the pAMPS is used as the osmotically active polymer block and pNIPAAM as the thermoresponsive block, comprising 16-22 wt.% total solid weight of the hydrogel formulation.

15. The composition according to any one of the preceding claims, further comprising the thermosensitive polymer and/or the osmotically active polymer as individual polymer.

16. The composition according to any one of the preceding claims, wherein the inorganic nanoparticles are ionically linked to the functional group in the block copolymer.

17. The composition according to any one of the preceding claims, wherein the functional group present in the polymer chains is chosen from the group consisting of hydroxyl, amine, epoxide, acid, anhydride, ammonium, carboxylate, sulphate, sulphonate, sulphinate, silane, phosphate, phosphonate, phosphinate, phosphite, phosphinite, sulphide, disulphide, maleic acid, phosphine, phosphine oxide, phosphonium, sulphonium, oxonium, ether, aldehyde and combinations of these.

18. The composition according to any one of the preceding claims, further comprising a biologically active ingredient.

19. The composition according to claim 18, wherein the biologically active ingredient is selected from the group consisting of drugs, proteins, cells, nucleotides, oligonucleotides, genes, and combinations thereof.

20. A method for the preparation of the hydrogel composition according to any one of claims 1-19, comprising combining the inorganic nanoparticles with the block copolymer in an aqueous solution at a temperature lower than the LCST of the copolymer.

21. A method for the preparation of an osmotically active gel, comprising heating the hydrogel composition according to any one of claims 1-19 to a temperature higher than the LCST of the copolymer.

22. A gel obtained by heating of the hydrogel composition according to any one of claims 1-19 to a temperature above the LCST of the block copolymer.

23. The use of the hydrogel composition according to any one of claims 1-19 or of the gel according to claim 22, in the sustained release of medicaments, tissue engineering and as a release agent of osmotic polymers.
